# EUROPEAN PATENT APPLICATION

(11) **EP 4 534 022 A1**
(43) Date of publication of application: **09.04.2025**
(21) Application number: 24204306.5
(22) Date of filing: 02.10.2024
(51) Int. Cl.: A61B 17/02, A61B 17/00, A61B 17/34

(54) **MEDICAL ELASTIC SHEET AND MEDICAL DEVICE USING THE SAME**

(30) Priority: 05.10.2023 JP 2023173380
(71) Applicant: Hakko Co., Ltd., Nagano 389-0806 (JP)
(72) Inventor: OTAGIRI, Daisuke, Chikuma-shi, 389-0806 (JP)
(74) Representative: Betten & Resch

(57) **Abstract**

A medical elastic sheet includes a plate-like base portion, and rib portions formed on at least one surface of the base portion, wherein the base portion comprises a plurality of cell regions that are each surrounded on an entire circumference by the rib portions and are continuously formed in plane through the rib portions, and wherein the rib portions are continuously connected to each other.

## Description

### CROSS-REFERENCE TO RELATED APPLICATIONS

The present application is based on Japanese patent application No. 2023-173380 filed on October 5, 2023, the entire contents of which are incorporated herein by reference.

### TECHNICAL FIELD

This disclosure relates to a medical elastic sheet and a medical device using the same.

### BACKGROUND OF THE INVENTION

In endoscopic surgery, medical devices such as wound retractors, dilators, and excluders are used to maintain the opening of the incision, to keep the natural opening dilated, and to compress and drain organs, etc. (see, for example, Patent Literatures 1, 2, and 3 )

The wound edge protection apparatus described in Patent Literature 1 has two rings arranged coaxially opposite each other and a tubular member (elastic sheet) made of silicone rubber fixed to the two rings with both end portions near the edges being extended. The excluder described in Patent Literature 2 has a ring-shaped frame and a plane-shaped sheet (elastic sheet) made of silicone rubber with a peripheral end being fixed to the frame.

The tissue retractor described in Patent literature 3 has an outer ring, an inner ring, and a flexible sheath (elastic sheet) made of abrasion and/or puncture resistant material that is fixed to the outer and inner rings with both end portions near the edges being extended. The flexible sheath is formed from multiple layers including a polymer film layer (e.g., a polyurethane layer) and a fabric layer (e.g., polyester woven fabric, metal components of metal fibers or strands), which allows the elasticity of the fabric layer to be modified in the lengthwise and circumferential directions by the fibers and twist weave of the fabric layer.

### Citation List

Patent Literature 1: Japanese Utility Model Registration No. 3062106
Patent Literature 2. Japanese Patent No. 5221447
Patent Literature 3: Japanese Patent No. 6247003

### SUMMARY OF THE INVENTION

Medical instruments include surgical instruments with sharp tips such as scissors, trocars, scalpels, suture needles, and syringes. In the wound edge protection apparatus described in Patent Literature 1, since the tubular member is formed from silicone rubber with a thin thickness, the sharp tip of the surgical instrument may contact and damage the tubular member. Since the flexible sheath is stretched in use, the tension may cause the damaged part to tear and widen, making it difficult to maintain the incision, or a part of the tubular member may break off and fall out into the body cavity, causing the remains to remain inside the body. The excluder device described in Patent Literature 2 has the same concerns as those described in Patent Literature 1.

According to the tissue retractors described in Patent Literature 3, depending on the material of the fabric layer combined with the polymer film layer in the flexible sheath, flexibility and elongation may be compromised, making protective patency of the incisional tissue more difficult than with a flexible sheath consisting only of a polymer film layer.

The object of the present invention is to provide a medical elastic sheet and a medical device using the same, which can suppress the expansion of a damage even if the elastic sheet is damaged by contact with a sharp tip of a medical device or the like, while maintaining protective patency to the body.

To solve the above problems, one aspect of the present invention provides: a medical elastic sheet, comprising:
a plate-like base portion; and
rib portions formed on at least one surface of the base portion,
wherein the base portion comprises a plurality of cell regions that are each surrounded on an entire circumference by the rib portions and are continuously formed in plane through the rib portions, and
wherein the rib portions are continuously connected to each other.

To solve the above problems, another aspect of the present invention provides: a medical device, comprising:
annular outer and inner rings arranged coaxially opposite each other, and
the elastic sheet with both end portions being fixed to the outer and inner rings.

To solve the above problems, a still another aspect of the present invention provides: a medical device, comprising:
an annular ring, and
the elastic sheet with a peripheral end portion being fixed to the ring.

### Advantageous Effects of the Invention

According to the present invention, it is possible to provide a medical elastic sheet and a medical device using the same, which can suppress the expansion of a damage even if the elastic sheet is damaged by contact with a sharp tip of a medical device or the like, while maintaining protective patency to the body.

### BRIEF DESCRIPTION OF DRAWINGS

FIG. 1 is a perspective view showing an example of the schematic configuration of a wound retractor in the first embodiment
FIG. 2 is a longitudinal cross-sectional view of the wound retractor shown in FIG. 1.
FIGS. 3A to 3C show an elastic sheet before the end portions are fixed to the outer and inner rings, in which FIG. 3A is a front view thereof, FIG. 3B is an enlarged view of part A shown in FIG. 3A, and FIG. 3C is a cross-sectional view along line B-B shown in FIG. 3A.
FIGS. 4A to 4E are diagrams showing the damage expansion control function of the elastic sheet.
FIG. 5A is a plan view of the wound retractor shown in FIG. 1, and FIG. 5B is a cross-sectional view along line C-C shown in FIG. 5A.
FIG. 6 is a front view of the wound retractor shown in FIG. 1.
FIG. 7A is a cross-sectional view along line D-D shown in FIG. 6 and FIG. 7B is a cross-sectional view along line E-E shown in FIG. 7A.
FIG. 8 is a cross-sectional view of an example of a state of use of the wound retractor in the first embodiment.
FIGS. 9A to 9E show a modified example 1 of the cell region.
FIGS. 9F is a front view of a modified example 2 of the cell region.
FIG. 9G is a cross-sectional view along line F-F in FIG. 9F.
FIG. 10 is a perspective view showing an example of the schematic configuration of a wound retractor in the second embodiment.
FIG. 11 is a longitudinal cross-sectional view of the wound retractor shown in FIG. 10.
FIG. 12 is a plan view of the wound retractor shown in FIG. 10.
FIG. 13 is a front view of the wound retractor shown in FIG. 10.
FIG. 14 is a perspective view showing an example of the schematic configuration of a wound retractor in third embodiment
FIG. 15A is a longitudinal cross-sectional view of the wound retractor shown in FIG. 14, and FIG. 15B is a longitudinal cross-sectional view of an example of a state of use of the wound retractor.
FIG. 16 is a plan view of an example of the schematic configuration of an excluder.
FIG. 17 is a front view of the excluder shown in FIG. 16.
FIG. 18 is a cross-sectional view along line G-G shown in FIG. 16.
FIGS. 19A to 19H are photographic images showing an example of damage experiments on an elastic sheet in Example.
FIGS. 20A to 20E are photographic images showing an example of damage experiments on an elastic sheet in Comparative example.

### DETAILED DESCRIPTION OF THE INVENTION

### [Embodiments]

Next, the embodiments of the present invention will be described in conjunction with appended drawings. In each drawing, components that have substantially the same functions will be marked with the same symbols and their redundant explanations will be omitted.

The development of medical devices such as wound retractors, dilators, and excluders has been directed toward increasing the strength of medical devices because if the elastic sheet used for them is partially damaged by the sharp tips of medical devices during treatment, the medical device may be damaged or partially detached and remain in the body if used as is. However, the inventor, through exchanging opinions with surgeons who use medical devices, converted the conventional idea of developing a medical device that is strong enough not to tear even if it is damaged, and achieved the present invention with the idea of developing a medical device that does not spread the damage even if a part of it is torn.

### [First Embodiment]

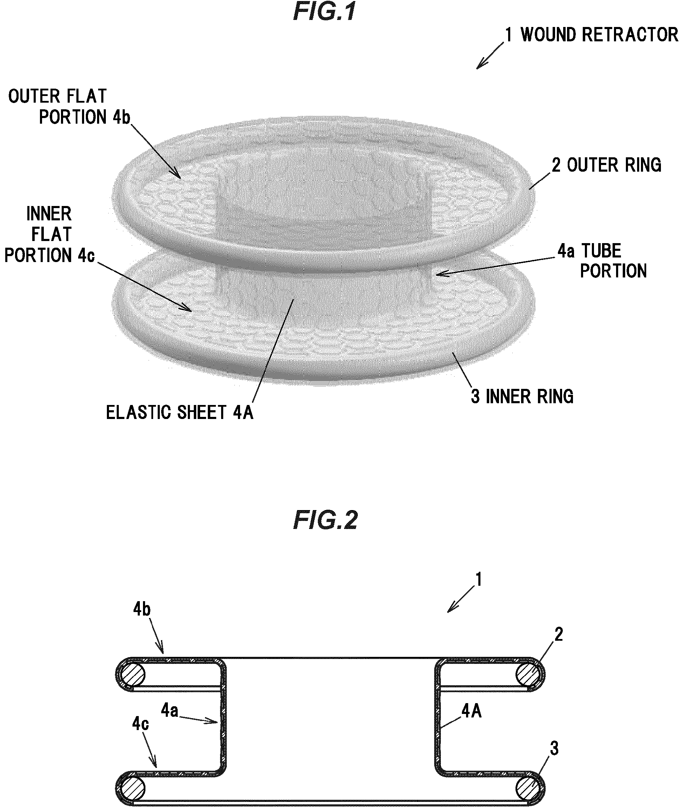

FIG. 1 is a perspective view showing an example of the schematic configuration of a wound retractor in the first embodiment.

FIG. 2 is a longitudinal cross-sectional view of the wound retractor shown in FIG. 1.

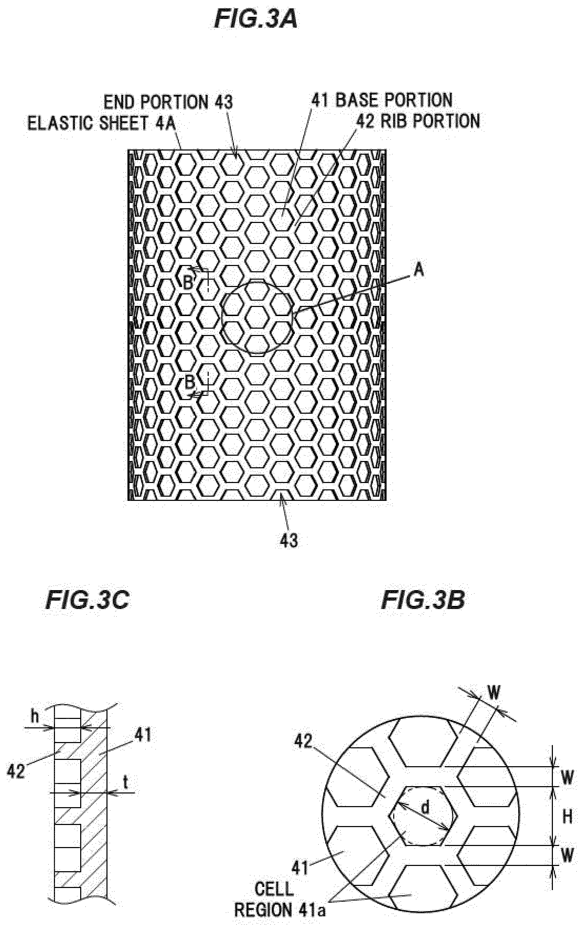

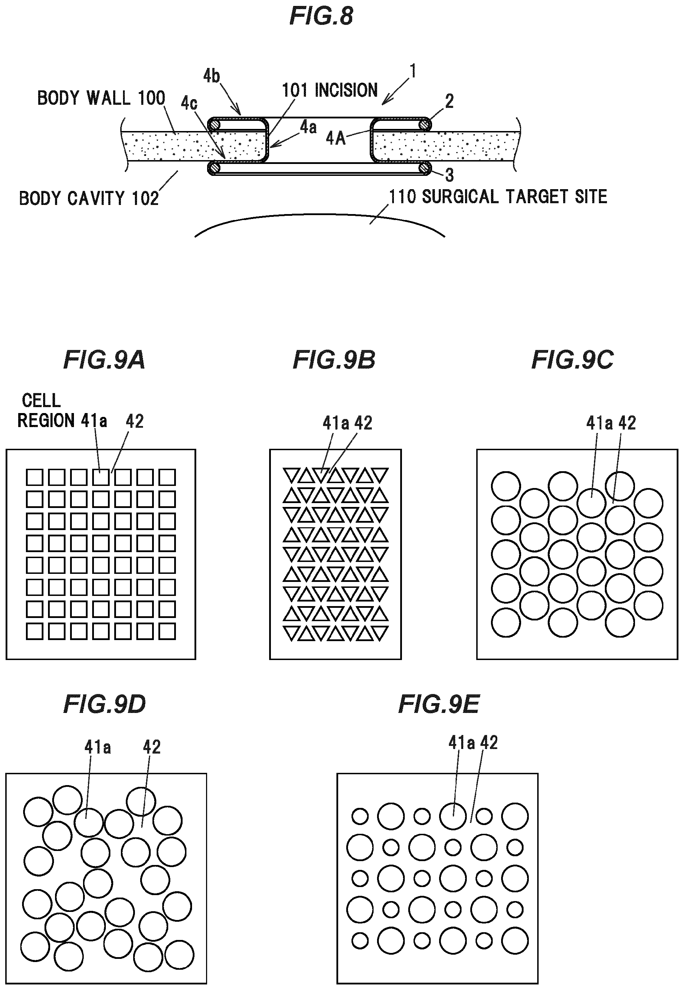

The wound retractor 1 in the first embodiment has an annular outer ring 2 and an annular inner ring 3 arranged coaxially opposite each other, and a tubular elastic sheet 4A with both end portions 43 (see FIG. 3, etc.) fixed to the outer ring 2 and the inner ring 3. The wound retractor 1 thus configured is, for example, attached to an incision 101 formed in a body wall 100 such as the abdominal wall of a living body (human, animal, etc.), as shown in FIG. 8. The wound retractor 1 is an example of a medical device. The elastic sheet 4A is an example of a medical elastic sheet.

The outer ring 2 and the inner ring 3 are placed outside and inside the body wall 100 through the incision 101, respectively, as shown in FIG. 8. The inner ring 3 is flexible enough to be inserted into a body cavity 102 through the incision 101 in an elliptical (i.e., oval) crushed state, and resilient enough to be restored to its original state (ring state) within the body cavity 102. The outer ring 2 has the same flexibility and restorative properties as the inner ring 3. Each of the outer ring 2 and the inner ring 3 has a circle diameter (hereinafter referred to as "outer diameter") that is larger than the length of the incision 101, for example.

The elastic sheet 4A is formed from a material that has flexibility to follow the shape of the incision 101 and elasticity to expand around the end portions 43 to be fixed to the outer ring 2 and the inner ring 3 in order to maintain the incision 101 in a protective manner The elastic sheet 4A has a function of inhibiting the expansion of damage (damage expansion control function) in the event that a sharp tip of a medical device or the like comes into contact with it and damages a part of it. Details of this damage expansion function are described below.

The elastic sheet 4A expands around the end portions 43 to cover the outer ring 2 and the inner ring 3, respectively, over an entire circumference, and then overlaps each end portion 43 of the elastic sheet 4A to form a bonding portion 43a, and fixes the bonding portions 43a to the outer ring 2 and the inner ring 3, respectively, by adhesion or welding to form a tube portion 4a with an inner diameter about 60 mm, an outer flat portion (i.e., outer flat plane portion) 4b with an outer diameter of about 100 mm, and an inner flat portion (i.e., inner flat plane portion) 4c with an outer diameter of about 100 mm Therefore, the outer flat portion 4b and the inner flat portion 4c are under tension

### (Configuration of elastic sheet)

FIGS. 3A to 3C show the elastic sheet 4A before the end portions 43 are fixed to the outer ring 2 and the inner ring 3, in which FIG. 3A is a front view thereof FIG. 3B is an enlarged view of part A shown in FIG. 3A, and FIG. 3C is a cross-sectional view along line B-B shown in FIG. 3A.

The elastic sheet 4A has a plate-like base portion 41 and a rib portion 42 formed on at least one side of the base portion 41 (in this case, the outer side of the tubular elastic sheet 4A, before the two end portions 43 are fixed to the outer ring 2 and the inner ring 3, forming a tubular shape, as shown in FIG. 3A.

When securing both the end portions 43 of the tubular elastic sheet 4A to the outer ring 2 and the inner ring 3, the areas near the end portions 43 of the elastic sheet 4A are extended to provide the bonding portions 43a where the end portions 43 are secured to the outer ring 2 and the inner ring 3, respectively.

The rib portion 42 is formed only on an outer surface of the tubular elastic sheet 4A in the present embodiment, but it may be formed only on an inner surface, or on the outer and inner surfaces. In the present embodiment, since the entire elastic sheet 4A is always exposed, it is preferable, that the cell regions described below, surrounded by the rib portion 42, are formed up to the end portion 43, but if the entire elastic sheet 4A is not always exposed (for example, in the third embodiment), the end portion 43 or the nearby region including the end portion 43, the cell regions may not be formed at the end portion 43 or in the surrounding area including the end portion 43.

The elastic sheet 4A is integrally formed from a resin material (e.g., silicone rubber, synthetic rubber, natural rubber, polyurethane, polyethylene, etc.) having elasticity such as flexibility and stretchability The tensile elongation rate of the material constituting the elastic sheet 4A is, for example, preferably 200% or more, and more preferably 4000/0 or more from the viewpoint of elasticity- The Shore A hardness (JIS K6253) of the material comprising the elastic sheet 4A is preferably, for example, A20 to A70 from the viewpoint of flexibility.

As shown in FIG. 3A, the base portion 41 has a lot of cell regions 41a, each fully enclosed (closed and enclosed) by the rib portion 42, being formed continuously in-plane, e.g., in two different directions (e.g., diagonally from bottom left to top right and from bottom right to top left in FIG. 3A). The rib portions 42 surrounding the cell regions 41a respectively are continuously connected to each other, e.g., in a mesh-like pattern.

The shape of the cell region 41a is hexagonal in the case shown in the drawings, but may be triangular, rectangular, circular (see FIGS. 9A to 9E), etc. When elongation is important, the cell region 41a may be made larger, and when tear resistance is important, the cell region 41a may be made smaller. The shape or area of the cell region 41a may be determined in such a manner that the flexibility or elasticity differs between the axial and circumferential directions. For example, since the outer flat portion 4b and the inner flat portion 4c are expanded to a greater extent than the tube portion 4a, the area of the cell region 41a of the outer flat portion 4b and the inner flat portion 4c may be larger than the area of the cell region 41a of the tube portion 4a. The area of the cell region 41a of the outer flat portion 4b and the inner flat portion 4c may be larger than the area of the cell region 41a of the side closer to the end portion 43 than the side closer to the tube portion 4a, because the side closer to the end portion 43 is more greatly expanded than the side closer to the tube portion 4a.

The arrangement direction of the cell regions 41a is not limited to the diagonal direction but may be in other directions such as axial and circumferential directions. The arrangement direction of the cell regions 41a may also be a direction according to the shape of the cell regions 41a. For example, if the cell region 41a is a square, the area of the rib portion 42 can be reduced by setting the arrangement direction of the cell regions 41a to the axial and circumferential directions. When comparing polygons with the same diameter of the circumscribed circle, by making the shape of the cell region 41a hexagonal, as in the present embodiment, the area of the cell region 41a can be wider than the area of other shapes (triangle, square, etc.), and the area of the rib portion 42 can be reduced, thus resulting in superior flexibility and elasticity. As a result, the elastic sheet 4A becomes more flexible and stretchable, and is less likely to be thickened when the elastic sheet 4A is rolled up (see the third embodiment below).

The axial size H of the cell region 41a shown in FIG. 3B and the diameter d of the circle inscribed in the cell region 41a are set in the present embodiment, for example, H = 3 mm and d = 3 mm, but the following values can be taken. That is, the axial size H of the cell region 41a (the diameter d of the inscribed circle) is preferably larger to maintain the flexibility of the elastic sheet 4A, for example, may be 1.5 mm or more, and even 2.5 mm or more may be used. The axial size H (the diameter d of the inscribed circle) of the cell region 41a is preferably smaller to suppress the expansion of damage in case a part of the elastic sheet 4A is damaged by contact with a sharp tip of a medical device or the like, for example, may be 20 mm or less, and even 10 mm or less or 7 mm or less.

The thickness t of the base portion 41 shown in FIG. 3C is, for example, t = 0.4 mm in the present embodiment, but the following values can be taken. That is, the thickness t of the base portion 41 is preferably smaller to maintain the flexibility of the elastic sheet 4A, for example, may be 2 mm or less, and even 1 mm or less. The thickness t of the base portion 41 is preferably larger to prevent the expansion of the damage described above, for example, may be 0.2 mm or more, and even 0.3 mm or more.

The width W of the rib portion 42 shown in FIG. 3B is, for example, W = 1 mm in the present embodiment, but the following values can be taken. That is, the width W of the rib portion 42 is preferably smaller to maintain the flexibility of the elastic sheet 4A, for example, may be 5 mm or less, and even 2 mm or less. The width W of the rib portion 42 is preferably larger to suppress the expansion of the damage described above, for example, may be 0.5 mm or more or 0.7 mm or more, and even 1.5 mm or more.

The height h of the rib portion 42 shown in FIG. 3C is, for example, h = 0.4 mm in the present embodiment, but the following values can be taken. That is, the height h of the rib portion 42 is preferably smaller to maintain the flexibility of the elastic sheet 4A, for example, may be 2 mm or less, and even 1 mm or less. The height h of the rib portion 42 is preferably larger to suppress the expansion of the damage described above, for example, may be 0.2 mm or more, and even 0.3 mm or more. The height h of the rib portion 42 may be made constant.

### (Damage, expansion control function of the elastic sheet)

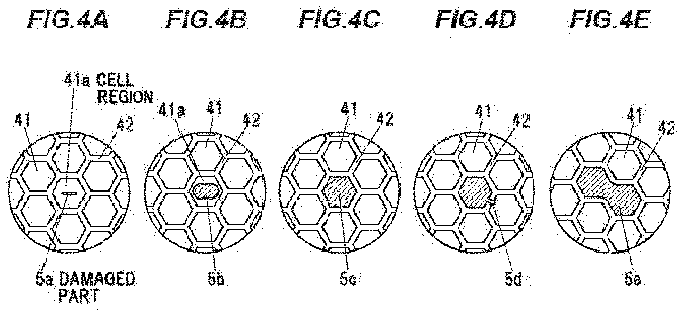

FIGS. 4A to 4E are diagrams showing the damage expansion control function of the elastic sheet 4A When the wound retractor 1 is attached to the incision 101 fanned on the body wall 100, tension is applied to the entire elastic sheet 4A. If a sharp tip of a medical instrument or the like contacts the base portion 41 of the elastic sheet 4A in such a state, causing a damaged part 5a. as shown in FIG. 4A, the damage may expand to a damaged part 5b shown in FIG. 4B and further to a damaged part 5c shown in FIG. 4C, but the rib portion 42 stops the tearing and inhibits the expansion of damage. However, the rib portion 42 stops the tearing and prevents the damage from expanding. Even if the rib portion 42 cracks as shown in FIG. 4D, the expansion can be controlled at a damaged part 5e in the adjacent cell region 41a, as shown in FIG. 4E. In other words, it is possible to avoid the damage being widely expanded by the rib portion 42, which makes it impossible to maintain incision 101, even when the elastic sheet 4A is under tension.

### (Configuration of the outer ring and inner ring)

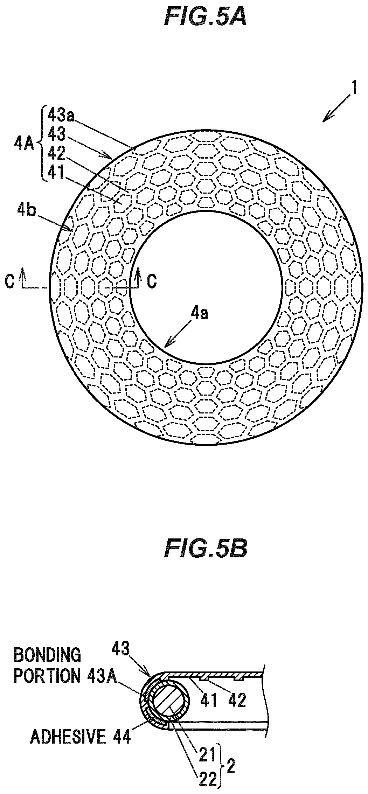

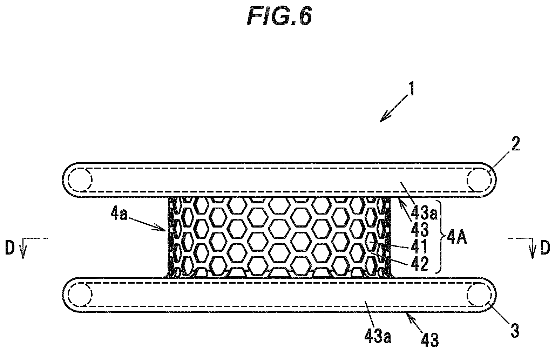

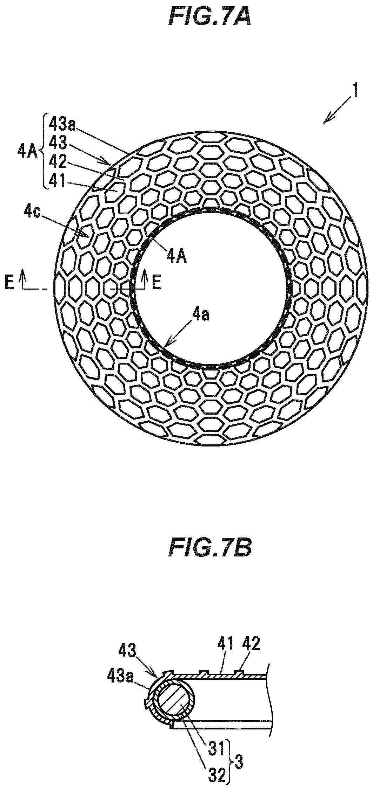

FIG. 5A is a plan view of the wound retractor 1 shown in FIG. 1, and FIG. 5B is a cross-sectional view along line C-C shown in FIG. 5A. FIG. 6 is a front view of the wound retractor 1 shown in FIG. 1. FIG. 7A is a cross-sectional view along line D-D shown in FIG. 6 and FIG. 7B is a cross-sectional view along line E-E shown in FIG. 7A.

The outer ring 2 has an annular core 21 and a cover tube 22 covering the surface of the core 21, as shown in FIG. 5B. The inner ring 3 has an annular core 31 and a cover tube 32 covering the surface of the core 31, as shown in FIG. 7B.

The cores 21 and 31 are formed from a flexible and resilient material, for example, a superelastic alloy such as Ni-Ti metal. By using superelastic alloy as the material of the core 31, as shown in FIG. 8, the inner ring 3 can be flexible enough to be inserted into the body cavity 102 through the incision 101 in an elliptical crushed state and restorable enough to be restored to its original state (ring state) in the body cavity 102.

If the end portions 43 of the elastic sheet 4A are directly fixed to the cores 21 and 31, when the cores 21 and 31 are deformed, the elastic sheet 4A may not absorb the deformation and the elastic sheet 4A may be damaged from the end portion 43 of the elastic sheet 4A. To prevent such damage to the elastic sheet 4A, the cores 21 and 31 are covered with the cover tubes 22 and 32, respectively, and the end portions 43 of the elastic sheet 4A are fixed to the cover tubes 22 and 32. The cover tubes 22 and 32 are formed from a resin such as silicone rubber, polyamide, and polyurethane, for example. In FIG. 5B, the end portion 43 of the elastic sheet 4A is illustrated being joined to the outer ring 2 by the adhesive 44 filled in the cell region 41a.

The outer diameters of the outer ring 2 and the inner ring 3 is preferably larger than the length of the incision 101. In particular, the outer diameter of the inner ring 3 is preferably a value according to the length of the incision 101 (e.g., 20 to 40 mm larger than the length of the incision 101) as shown in Table 1. The inner diameter of the tube portion 4a of the elastic sheet 4A is preferably a value according to the length of the incision 101 (e.g., a value equal to or 10 to 40 mm smaller than the length of the incision 101) as shown in Table 1. The thicknesses of the outer ring 2 and the inner ring 3 are, for example, 5 mm in diameter in the present embodiment, but may be 3 to 6 mm. The thicknesses of the outer ring 2 and the inner ring 3 need not be the same. For example, by making the inner ring 3 thinner than the outer ring 2, it is easier to crush the inner ring 3 into an elliptical shape when it is placed on the incision 101, and by making the inner ring 3 thicker than the outer ring 2, it is harder to pull out of the incision 101.

**[Table 1]**

| Length of incision | Outer diameter of inner ring | Inner diameter of tube portion of elastic sheet | Spacing between outer ring and inner ring |
|---|---|---|---|
| 10 to 20 mm | Up to 40 mm | 15 to 20 mm | Equal to or less than the thickness of the body wall (e.g., 10 to 35 mm) |
| 20 to 40 mm | Up to 70 mm | Approx. 35 mm | |
| 30 to 60 mm | Up to 100 mm | Approx. 60 mm | |
| 50 to 80 mm | Up to 120 mm | Approx. 60 mm | |
| 80 to 120 mm | Up to 150 mm | Approx. 80 mm | |

The distance between the outer ring 2 and the inner ring 3 is preferably selected to be equal to or less than the thickness of the body wall 100. This makes it easier for the elastic sheet 4A to extend and adhere to the body wall 100. The outer diameter of the outer ring 2 is set to be equal to the outer diameter of the inner ring 3 in the present embodiment, but it may be a different value (e.g., a value larger than the outer diameter of the inner ring 3).

### (Manufacturing method)

Next, an example of a manufacturing method for the wound retractor 1 will be described. First, an outer ring 2, an inner ring 3, and a tubular elastic sheet 4A are prepared. The tubular elastic sheet 4A is manufactured, for example, by injection molding liquid silicone rubber. The tubular elastic sheet 4A may also be produced by compression molding of millable silicone rubber. The surface of the elastic sheet 4A becomes smooth by injection molding. The outer ring 2 and the inner ring 3 are manufactured, for example, by covering cores 21 and 31 with cover tubes 22 and 32 made of silicone rubber, respectively. Next, the tubular elastic sheet 4A is expanded around end portions 43 to cover the outer ring 2 and the inner ring 3 all around (i.e., over entire circumferences, respectively), and the end portions 43 are bonded to the outer ring 2 and the inner ring 3 by adhesion, respectively, In this way, the wound retractor 1 is manufactured. The elastic sheet 4A may be prepared as a tubular elastic sheet material of a length that allows multiple pieces to be taken, and cut out from the elastic sheet material as needed.

### (Example of use of a wound retractor)

FIG. 8 is a cross-sectional view of an example of a state of use of the wound retractor 1 in the first embodiment. In this drawing, the cell regions 41a are omitted. After forming an incision 101 in a body wall 100 of a human, animal, or other living body, the wound retractor 1 is inserted into a body cavity 102 through the incision 101 with the inner ring 3 of the wound retractor 1 crushed into an elliptical shape, and the wound retractor 1 is attached to the incision 101. Since the inner diameter of the tube portion 4a of the wound retractor 1 is about the same as the length of the incision 101 or 10 to 40 mm smaller, the tube portion 4a adheres closely to the incision 101. Thereafter, an internal insertion device (surgical instrument, endoscope, etc.) is inserted into the body cavity 102 through the tube portion 4a, and surgery, observation, etc. are performed on a surgical target site 110.

### [Modified Example 1]

FIGS. 9A to 9E shows a modified example 1 of the cell region 41a. In the present embodiment, the shape of the cell region 41a is hexagonal, but various variations are possible. For example, the shape of the cell region 41a can be a rectangle as shown in FIG. 9A. The shape of the cell region 41a may also be triangular or circular as shown in FIGS. 9B and 9C. The direction of arrangement of the cell regions 41a is not limited to two different directions from each other, but may be a random arrangement as shown in FIG. 9D. The size of the cell regions 41a may be various sizes, as shown in FIG. 9E, or may be randomly arranged and may be different sizes of the cell regions 41a. The shape of the cell region 41a is not limited to the above and may be arbitrary. For example, in addition to hexagons, it may be pentagons, octagons, or other polygons such as quadrilaterals or larger, floral patterns, ovals, trapezoids, etc. The shape of the cell region 41a can be applied to other forms as described below.

### [Modified example 2]

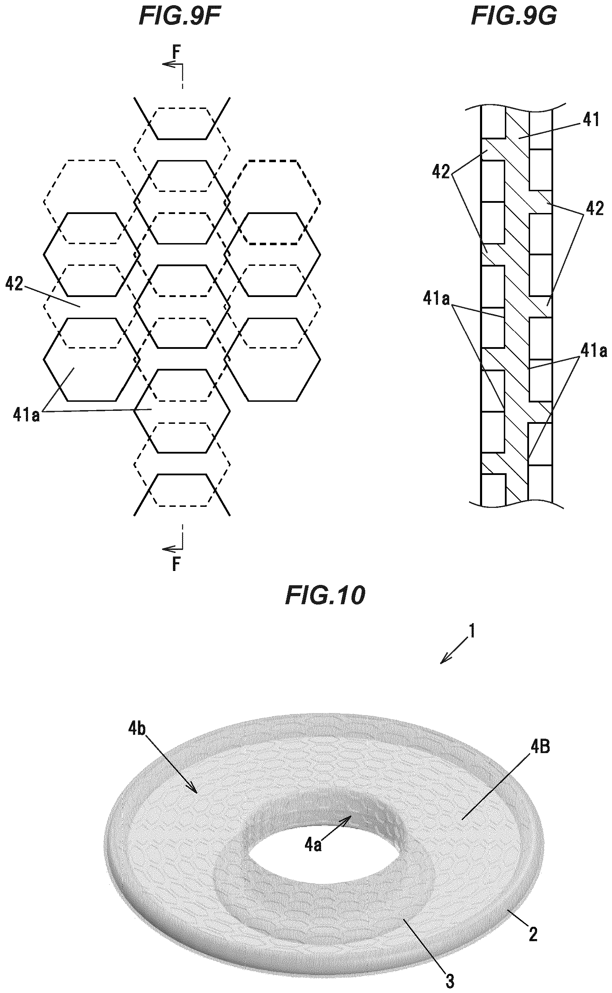

FIGS. 9F is a front view of a modified example 2 of the cell region 41a. FIG. 9G is a cross-sectional view along line F-F in FIG. 9F. In the present embodiment, the cell region 41a is provided on only one side of the base portion 41, but as shown in FIGS. 9F and 9G, it may be provided on both sides of the base portion 41. In this case, as shown in FIGS. 9F and 9G, it is preferable to provide the cell regions 41a on one side and the cell regions 41a on the other side at a predetermined distance (e.g., half pitch) from each other to enhance the damage expansion control function. The modified example 2 can also be applied to other embodiments as described below.

### (The effects of the present embodiment)

The following effects are achieved by the present embodiment.
(a) Since the elastic sheet 4A is formed from a flexible and elastic material, it can maintain its protective patency to the body. In addition, since the elastic sheet 4A has a damage expansion control function, even if the elastic sheet 4A is damaged due to contact with a sharp tip of a medical device or the like, the expansion of the damage can be controlled.
(b) Since the elastic sheet 4A is formed from a single resin material, the material cost can be reduced compared to elastic sheets made from multiple materials. In addition, since the manufacturing process is reduced, a low-cost elastic sheet can be provided.
(c) Because the rib portion 42 is provided on the outer surface of the base portion 41 of the tube portion 4a, the rib portion 42 presses against the body wall, thereby inhibiting displacement against the incision 101.

Each of the outer ring 2 and the inner ring 3 may have an elliptical annular shape (i.e., oval ring shape), and the elastic sheet 4A may be formed into an elliptical cylinder. This allows a large operating space to be obtained by attaching the outer ring 2 and the inner ring 3 to the small linear incision 101 so that the direction perpendicular to the small linear incision 101 is the major axis of the ellipse of each of the outer ring 2 and the inner ring 3. In addition, when attached along the intercostal space, it conforms to the intercostal gap and allows for a comfortable wound opening, significantly reducing postoperative intercostal pain.

### [Second Embodiment]

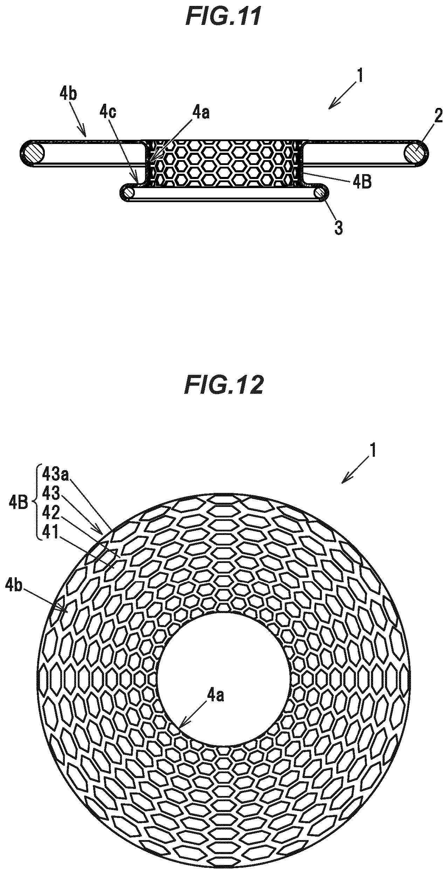

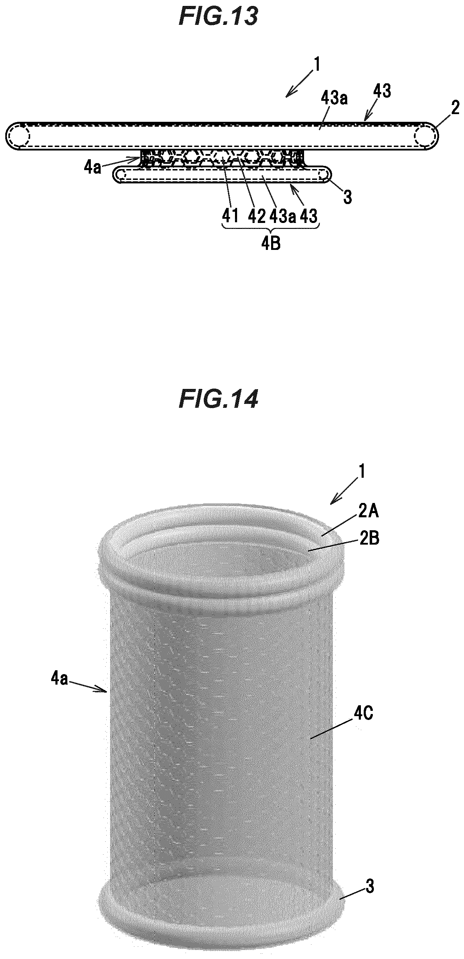

FIG. 10 is a perspective view showing an example of the schematic configuration of a wound retractor 1 in the second embodiment. FIG. 11 is a longitudinal cross-sectional view of the wound retractor 1 shown in FIG. 10. FIG. 12 is a plan view of the wound retractor 1 shown in FIG. 10. FIG. 13 is a front view of the wound retractor 1 shown in FIG. 10.

In the first embodiment, the rib portions 42 were formed on the outer surface of the tubular elastic sheet 4A, but in the present embodiment, the rib portions 42 are formed on the inner surface of a tubular elastic sheet 4B. In the first embodiment, the outer diameter of the inner ring 3 was equal to the outer diameter of the outer ring 2, but in the present embodiment, the outer diameter of the inner ring 3 is smaller than the outer diameter of the outer ring 2 and the thickness of the inner ring 3 is thinner than the thickness of the outer ring 2, and the rest of the configuration is similar to the first embodiment. The following explanation focuses on the points that differ from the first embodiment. Since the wound retractor 1 in the second embodiment is manufactured in the same way as the first embodiment, the description of the manufacturing method is omitted.

As shown in FIGS. 10 to 13, the wound retractor 1 in the second embodiment has, as in the first embodiment, an annular outer ring 2 and an annular inner ring 3 arranged coaxially opposite each other, and a tubular elastic sheet 4B with both end portions 43 fixed to the outer ring 2 and the inner ring 3, respectively. The end portions 43 of the elastic sheet 4B are fixed to the outer ring 2 and the inner ring 3 by adhesion or welding to form bonding portions 43a, respectively. The elastic sheet 4B is an example of a medical elastic sheet.

The elastic sheet 4B has the same damage expansion control function as the first embodiment, and has a plate-like base portion 41 and rib portions 42 formed on at least one side of the base portion 41 (in the present embodiment, the inner surface of the tubular elastic sheet 4B). The elastic sheet 4B is tubular before the end portions 43 are fixed to the outer ring 2 and the inner ring 3, respectively. Since the wound retractor 1 is used, for example, in surgeries in which tissues are easily damaged, such as lymph node dissection of the breast line, the elastic sheet 4B may be thinner than in other embodiments (for example, the base portion 41 is about 0.3 mm thick and the rib portion 42 is about 0.3 mm high) to avoid stressing the tissues.

The outer ring 2 has an annular core 21 and a cover tube 22 covering the surface of the core 21, as in the first embodiment. The inner ring 3, like the outer ring 2, has an annular core 31 and a cover tube 32 covering the surface of the core 31. However, the inner ring 3 is thinner than the outer ring 2, and the outer diameter of the inner ring 3 is smaller than the outer diameter of the outer ring 2. The outer ring 2 and the inner ring 3 shall have an elliptical annular shape, and the elastic sheet 4B may be formed into an elliptical cylinder.

According to the second embodiment, the same effects as the first embodiment are achieved, and the following effects are also achieved. That is, since rib portions 42 are provided on the inner surface of the base portion 41 of the tube portion 4a, the contact area on the inner surface of the tube portion 4a is reduced and the frictional force against the surgical instrument can be reduced, which is expected to suppress damage to the elastic sheet 4B. In addition, since the inner ring 3 is formed thinner than the outer ring 2 and the outer diameter of the inner ring 3 is smaller than the outer diameter of the outer ring 2, it is easier to insert the inner ring 3 into the body cavity 102 through the incision 101 in an elliptical crushed state.

### [Third Embodiment]

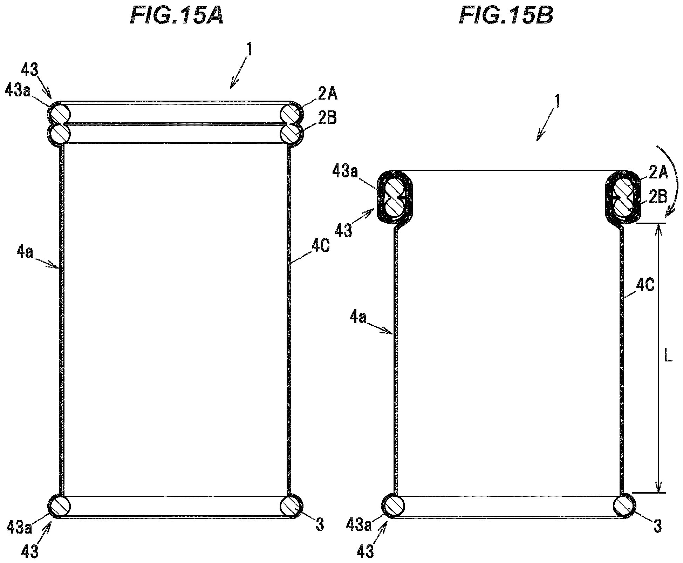

FIG. 14 is a perspective view showing an example of the schematic configuration of a wound retractor in third embodiment. FIG. 15A is a longitudinal cross-sectional view of the wound retractor shown in FIG. 14, and FIG. 15B is a longitudinal cross-sectional view of an example of a state of use of the wound retractor.

The first embodiment has a tube portion 4a, an outer flat portion 4b, and an inner flat portion 4c. The third embodiment has a tube portion 4a and allows the upper part of an elastic sheet 4C to be rolled up without the outer flat portion 4b and the inner flat portion 4c, and the rest is constructed in the same way as the first embodiment. The following explanation focuses on the points that differ from the first embodiment.

The wound retractor 1 in the third embodiment has a pair of annular outer rings 2A, 2B and an inner ring 3 arranged coaxially opposite each other, and the tubular elastic sheet 4C with both end portions 43 being fixed to the outer rings 2A, 2B and the inner ring 3 to form a tubular tube portion 4a. The end portions 43 of the elastic sheet 4C are fixed to the outer rings 2A, 2B and the inner ring 3 by adhesion or welding to form bonding portions 43a. The elastic sheet 4C is an example. of a medical elastic sheet.

The elastic sheet 4C has the same damage expansion control function as the first embodiment, and consists of a plate-like base portion 41, rib portions 42 formed on at least one side of the base portion 41 (in the present embodiment, the outer surface of the tubular elastic sheet 4C), and end portions 43 fixed to the outer rings 2A, 2B and the inner ring 3. Since the wound retractor 1 in the present embodiment does not have the outer flat portion 4b and the inner flat portion 4c as in the first embodiment, it is not necessary for the elastic sheet 4C to expand around the end portion 43 to elasticity to secure it to the ring. The elastic sheet 4C is tubular before the end portions 43 are secured to the outer rings 2A, 2B and the inner ring 3.

The axial length of the elastic sheet 4C is preferably in accordance with the outer diameters of the outer rings 2A and 2B to adapt to the thickness of the body wall 100 after several windings, e.g., about 100 mm if the outer diameter of the outer rings 2A and 2B is 70 mm and about 150 mm if the outer diameter of the outer rings 2A and 2B is 100 mm.

The pair of outer rings 2A and 2B are formed from an elastic material (e.g., silicone rubber, polyurethane, etc.) and do not have a core 21 as in the first embodiment. The outer rings 2A and 2B may be composed of a core made of a superelastic alloy or the like and a cover tube covering the core, as in the first embodiment.

The pair of outer rings 2A and 2B are either molded together or joined by adhesion or fusion along the axial direction of the wound retractor 1. This allows the resilience of the elastic sheet 4C to be maintained when it is wound up, and prevents unwinding. The outer rings 2A and 2B may be in the form of two rings integrated together (e.g., a flat elliptical shape long in the axial direction of the wound retractor 1).

The inner ring 3 is formed from an elastic material (e.g., silicone rubber, polyurethane, etc.) and is not provided with a core 31 as in the first embodiment. The inner ring 3 may have an annular core 31 and a cover tube 32 covering the surface of the core 31, as in the first embodiment. The outer diameter of the inner ring 3 may be equal to or different from the outer diameter of the pair of outer rings 2A and 2B.

According to the third embodiment, it has the same effects as the first embodiment and also has the following effects. As shown in FIG. 15B, the distance L between the outer ring 2B and the inner ring 3 can be adjusted by winding up the upper part of the elastic sheet 4C (a pair of outer rings 2A and 2B) in the direction indicated by the arrow in the same drawing, depending on the area of the body where the body is worn, in other words. Since the axial length of the tube portion 4a can be adjusted by changing the number of times it is wound up according to the thickness of the body wall 100, fewer types of product standards are required. Furthermore, since there is no need to forcibly stretch the elastic sheet 4C when attaching the incision 101, cracks are less likely to spread. The direction in which the pair of outer rings 2A and 2B are rotated when rolled up can be the opposite of the direction shown in FIG. 15B.

The outer rings 2A, 2B and the inner ring 3 may have an elliptical ring shape, and the elastic sheet 4C may be formed into an elliptical cylinder. The area near the end portion 43 of the elastic sheet 4C may consist of only the base portion 41 without the cell region 41a. In this case, it is easier to roll up the elastic sheet 4C and the elastic sheet 4C is less likely to be thick when rolled up.

### [Fourth Embodiment]

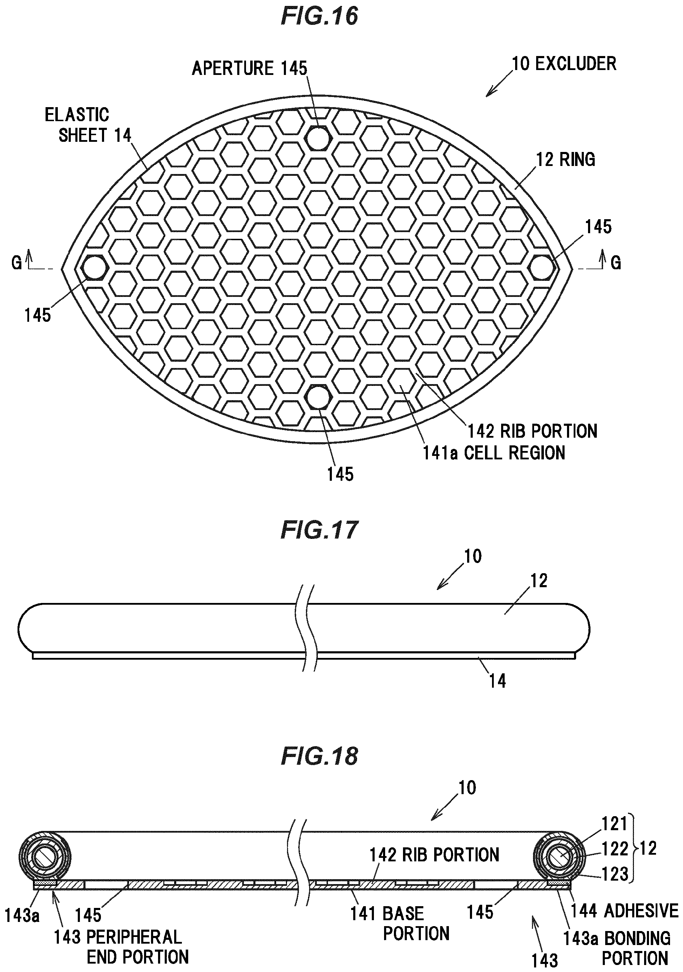

FIG. 16 is a plan view of an example of the schematic configuration of an excluder. FIG. 17 is a front view of the excluder shown in FIG. 16. FIG. 18 is a cross-sectional view along line G-G shown in FIG. 16.

An excluder 10 in the fourth embodiment has a substantially elliptical shape similar to a rugby ball shape with a major axis of 120 mm and a minor axis of 80 mm, for example, and includes a ring 12 having a substantially elliptical annular shape (including an elliptical annular shape), and an elastic sheet 14 with the peripheral end portion 143 being fixed to the ring 12. For example, the excluder 10 is used to protectively exclude organs such as the lungs (i.e., temporarily push out and eliminate objects such as organs or tumors that obstruct surgical operations or the surgical field). The excluder 10 is an example of medical devices. An elastic sheet 14 is an example of medical elastic sheets.

As shown in FIG. 18, the ring 12 includes a core 121 with a substantially elliptical annular shape (including an elliptical annular shape), a first cover tube 122 covering a surface of the core 121, and a second cover tube 123 covering the first cover tube 122. Here, the second cover tube 123 may not be provided.

The core 121 is formed from a flexible and resilient material, for example, a superelastic alloy such as Ni-Ti based metal, as in the first embodiment.

The first cover tube 122 and the second cover tube 123 are formed from resin, such as silicone rubber, polyamide, polyurethane, etc., as in the first embodiment.

The elastic sheet 14 is formed from a flexible and elastic material as in the first embodiment. The elastic sheet 14 is fixed to the ring 12 by adhesion or welding at the peripheral end portion 143.

The elastic sheet 14 has the same damage expansion control function as the first embodiment, and as shown in FIGS. 16 and 18, includes a base portion 141 having a substantially elliptical shape (including an elliptical annular shape) and formed in a flat plane shape, a rib portion 142 formed on at least one side of the base portion 141 (in the present embodiment, the upper surface), a peripheral end portion 143 where the plate-like base portion 41 is fixed to the ring 12, and a plurality of openings 145 into which threads or instruments are inserted. The peripheral end portion 143 of the elastic sheet 14 is fixed to the ring 12 by adhesion or fusion to form a bonding portion 143a. In FIG. 18, the peripheral end portion 143 of the elastic sheet 14 is shown joined to the ring 12 by an adhesive 144 filled in a cell region 141a.

As in the first embodiment, the base portion 141 has a series of cell regions 141a surrounded by rib portions 142 in two different directions from each other (e.g., diagonally from bottom left to top right and from bottom right to top left in FIG. 16), as shown in FIG. 16. The shape of the cell region 141a is hexagonal, as in the first embodiment, but may be any other shape. The rib portion 142 may be formed only on a bottom surface of the base portion 141, or may be formed on the top and bottom surfaces of the base portion 141.

### (Manufacturing method)

Next, an example of a manufacturing method of the excluder 10 will be described. First, a ring 12 and a flat (planar) elastic sheet 14 are prepared. The flat elastic sheet 14 is manufactured, for example, by injection molding liquid silicone rubber. The flat elastic sheet 14 may also be produced by compression molding millable silicone rubber. The surface of the elastic sheet 14 becomes smooth by injection molding. The ring 12 is manufactured, for example, by covering the core 121 with a first cover tube 122 and a second cover tube 123 made of silicone rubber. The peripheral end portion 143 of the flat elastic sheet 14 is then bonded to the ring 12 by adhesion or welding. In this manner, the excluder 10 is manufactured. The elastic sheet 14 may be prepared from a flat elastic sheet material of a size that allows multiple pieces to be taken, and cut out from the elastic sheet material as needed.

According to the fourth embodiment, since the excluder 10 has a substantially elliptical shape, it can be easily reduced in diameter when inserted into the body cavity 102. In addition, since the elastic sheet 14 has a damage expansion control function, even if the elastic sheet 14 is damaged by contact with a sharp tip of a medical device or the like, the expansion of damage can be controlled.

The shape of the elastic sheet 14 is arbitrary, but may correspond to the annular shape of the ring 12. For example, the ring 12 may have an annular shape, and the elastic sheet 14 may be flat, such as circular, rectangular, or polygonal. The elastic sheet 14 may be prepared from a flat elastic sheet material of a size that allows multiple pieces to be taken, and cut out from the elastic sheet material as needed.

### [Examples]

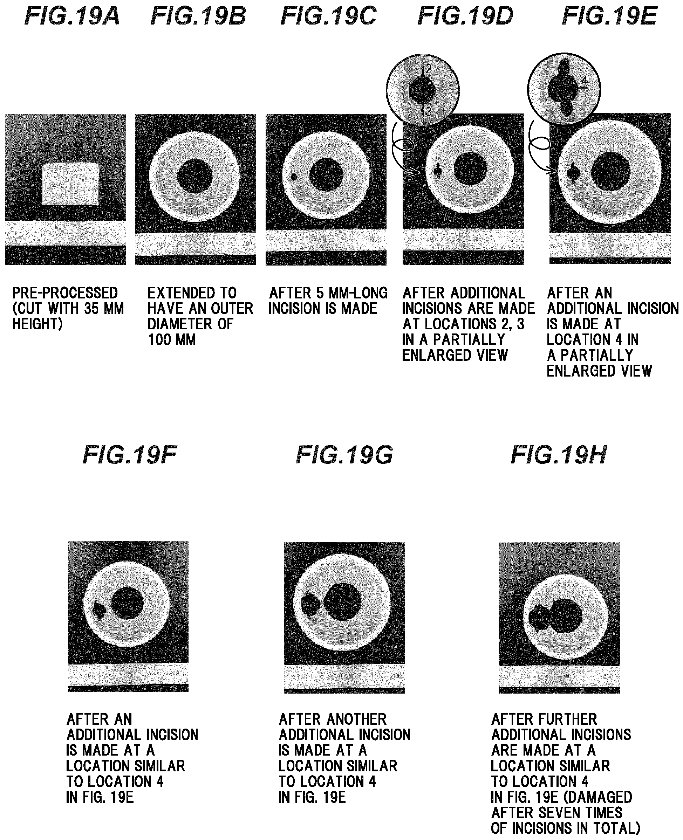

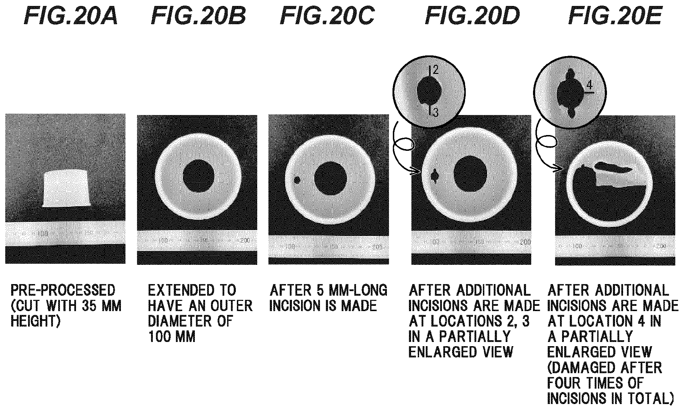

Next, referring to FIGS. 19A to 19H, and 20A to 20E, experimental examples of damage to elastic sheets in Example and comparative example. FIGS 19A to 19H are photographic images showing an example of damage experiments on an elastic sheet 4A in Example. FIGS. 20A to 20E are photographic images showing an example of damage experiments on an elastic sheet in comparative example.

### (Experimental sample in Example)

The experimental sample in Example correspond to the first embodiment, and the following sample was used. As to the size of the wound retractor 1, the inner diameter of the tube portion 4a was 35 mm and the outer diameter thereof was 100 mm As to the physical properties of the material of the elastic sheet 4A, silicone rubber with a tensile elongation of 285% and a hardness of A30 was used. As to the size of the cross-sectional shape of the elastic sheet 4A, the width W of the rib portion 42 was 1 mm the thickness t of the base portion 41 was 0.4 mm, and the height h of the rib portion 42 was 0.4 mm. As to the shape and size of cell region 41a, tthe shape of cell region 41a was hexagonal, the axial size H of the cell region 41a was 3 mm, and the diameter d of the circle inscribed in the cell region 41a was 3 mm

### (Experimental sample in Comparative example)

The following experimental sample was used in the comparative example. As to the size of the wound retractor, the inner diameter of the tube portion was 35 mm and the outer diameter thereof was 100 mm As to the physical properties of the elastic sheet, silicone rubber with a tensile elongation of 285% and a hardness of A30 was used. As to the size of the cross-sectional shape of the elastic sheet, it was 1 mm thick without rib portions.

### (Experimental results of Example)

As a pre-processing (i.e., pretreatment), the upper side of the tubular elastic sheet 4A shown in FIG. 3A was cut so that its axial length (height) was 35 mm, as shown in FIG. 19A. Next, as shown in FIG. 19B, the elastic sheet 4A was extended and fixed near the end portion 43 of the elastic sheet 4A to the inner ring 3 with an outer diameter of 100 mm.

Next, as shown in FIG. 19C, a 5 mm-long incision was made in the inner flat portion 4c to simulate the damage caused by the sharp tip of the surgical instrument (the same is shown below). The damaged part was formed by making the 5 mm long incision in the inner flat portion 4c. Next, as shown in FIG. 19D, 5 mm-long incisions were made on both sides of the damaged part (at the locations indicated by 2 and 3 in a partially enlarged view). Next, as shown in FIG. 19E, a 5 mm-long incision was made in the center of the damaged part (at the location indicated by 4 in a partially enlarged view). Next, as shown in FIGS. 19F and 19G, a 5 mm-long incision was made in the center of the damaged part (at the location indicated by 4 in a partially enlarged view). At this stage, the damaged part did not expand significantly, but when a 5 mm-long incision was made again in the center of the damaged part (at the location indicated by 4 in a partially enlarged view), the crack expanded from the damaged part to the opening in the center, as shown in FIG. 19H. In other words, the elastic sheet 4A was damaged by seven times of incisions in total

### (Experimental results of Comparative example)

As a pre-processing, the upper side of the tubular elastic sheet shown in FIG. 20A was cut so that the axial length (height) of the sheet was 35 mm Next, as shown in FIG. 20B, the elastic sheet was extended and fixed near the end of the elastic sheet to the inner ring 3 with an outer diameter of 100 mm

Next, as shown in FIG. 20C, a 5 mm-long incision was made in the inner flat portion to form the damaged portion. Next, as shown in FIG. 20D, 5 mm-long incisions were made on both sides of the damaged part (at the locations indicated by 2 and 3 in a partially enlarged view). At this stage, the damaged part did not expand significantly, but when a 5 mm-long incision was made in the center of the damaged part (at the location indicated by 4 in a partially enlarged view), the crack expanded from the damaged part to the opening in the center, as shown in FIG. 20E. In other words, the elastic sheet was damaged by four times of incisions in total.

### (Summary of experimental results)

Despite the fact that the overall thickness of the elastic sheet 4A in Example was 0.8 mm (0.4 mm for the base portion and 0.4 mm for the rib portion), which is thinner than the 1 mm thickness of the elastic sheet in Comparative example, the experimental sample was damaged after seven times of incisions and the experimental sample in Comparative example was damaged after four times of incisions. The reason that the number of times of incisions leading up to the breakage of the elastic sheet was higher in Example than in Comparative example is assumed to be due to the damage expansion control function of the elastic sheet 4A in Example

The following inventions may be ascertained from this disclosure.
[1] A medical elastic sheet having a plate-like base portion and rib portions formed on at least one surface of the base portion, wherein the base portion has a plurality of cell regions that are each surrounded on an entire circumference by the rib portions and are continuously formed in plane through the rib portions, and the rib portions are continuously connected to each other.
[2] The above cell region may have a triangular, polygonal, circular, or elliptical shape.
[3] The above cell region may have a hexagonal shape.
[4] The base portion and rib portions may be formed from the same resin material.
[5] In the medical elastic sheet of any one of [1] to [4], the base portion may be formed in a tubular shape and the rib portions may be formed on at least one of the outer and inner surfaces of the tubular base portion.
[6] In the medical elastic sheet of any of [1] to [4], the base portion may be formed in a flat shape.
[7] A medical device having annular outer and inner rings arranged coaxially opposite each other, and the elastic sheet as described in [5] with both end portions being fixed to the outer and inner rings.
[8] A medical device having an annular ring, and the elastic sheet as described in [6] with a peripheral end portion being fixed to the ring.

According to the medical elastic sheet and medical device configured as described above, the following effects are achieved.

According to the invention described in [1], even if the elastic sheet is damaged by contact with the sharp tip of a surgical instrument such as a medical instrument, the expansion of damage can be suppressed, while maintaining the protective patency to the body.

According to the invention described in [2], the cell region is simple in shape, making it easier to surround the cell region with the rib portions.

According to the invention described in [3], when compared with the same diameter of the circumscribed circle, the area of the cell region is larger than other shapes, resulting in superior flexibility and elasticity.

According to the invention described in [4], it is possible to provide inexpensive medical elastic sheets because the material cost can be kept low compared to medical elastic sheets using multiple materials and the manufacturing process is reduced.

According to the inventions described in [5] and [7], a ring can be attached to both end portions of a tubular medical elastic sheet to use it as a wound retractor for medical devices.

According to the inventions described in [6] and [8], by attaching a ring to the peripheral end portion of a flat medical elastic sheet, the sheet can be used as an excluder for medical devices.

The above-described embodiments of the invention are not limited to the above embodiments but can be varied and implemented in various ways. For example, although the above embodiments describe a wound-retractor and a pressure-discharge device, the present invention can be applied to other medical devices such as a dilator for maintaining the natural opening in a dilated state.

Some of the components of the above embodiments may be omitted or changed. In addition, processes may be added, deleted, changed, or replaced in the use of the above embodiments.

## Claims

1. A medical elastic sheet, comprising:
a plate-like base portion; and
rib portions formed on at least one surface of the base portion,
wherein the base portion comprises a plurality of cell regions that are each surrounded on an entire circumference by the rib portions and are continuously formed in plane through the rib portions, and
wherein the rib portions are continuously connected to each other.

2. The medical elastic sheet, according to claim 1, wherein the cell region comprises a triangular, polygonal, circular, or elliptical shape.

3. The medical elastic sheet, according to claim 2, wherein the cell region comprises a hexagonal shape.

4. The medical elastic sheet, according to claim 1, wherein the base portion and the rib portions are formed from a same resin material.

5. The medical elastic sheet, according to any one of claims 1 to 4, wherein the base portion is formed in a tubular shape and the rib portions are formed on at least one of outer and inner surfaces of the tubular base portion.

6. The medical elastic sheet, according to any one of claims 1 to 4, wherein the base portion is formed in a flat shape.

7. A medical device, comprising:
annular outer and inner rings arranged coaxially opposite each other, and
the elastic sheet according to claim 5 with both end portions being fixed to the outer and inner rings.

8. A medical device, comprising:
an annular ring, and
the elastic sheet according to claim 6 with a peripheral end portion being fixed to the ring.
